(19) Europäisches Patentamt / European Patent Office / Office européen des brevets

(11) **EP 3 498 253 B1**

(12) **EUROPEAN PATENT SPECIFICATION**

(45) Date of publication and mention of the grant of the patent:
**17.02.2021 Bulletin 2021/07**

(21) Application number: **17207391.8**

(22) Date of filing: **14.12.2017**

(51) Int Cl.:
**A61K 8/14** *(2006.01)*          **C11D 3/50** *(2006.01)*
**A61Q 19/10** *(2006.01)*          **A61Q 13/00** *(2006.01)*
**C11D 1/722** *(2006.01)*          **A61K 8/37** *(2006.01)*
**A61K 8/39** *(2006.01)*          **C11D 1/83** *(2006.01)*
**C11D 3/00** *(2006.01)*          **C11D 17/00** *(2006.01)*

(54) **VESICLES FOR DELAYED DELIVERY OF FRAGRANCE THEIR PREPARATION AND USE THEREOF**

VESIKEL ZUR VERZÖGERTEN ABGABE EINES DUFTSTOFFES, DEREN HERSTELLUNG UND VERWENDUNG DAVON

VÉSICULES DE REMISE DIFFÉRÉE DE PARFUMS, LEUR PRÉPARATION ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.06.2019 Bulletin 2019/25**

(73) Proprietor: **Clariant International Ltd**
**4132 Muttenz (CH)**

(72) Inventors:
• **DAHMS, Gerd**
**47138 Duisburg (DE)**

• **WAGDARE, Nagesh Appasaheb**
**Airoli, Navi Mumbai 400 708 (IN)**

(74) Representative: **Paczkowski, Marcus**
**Clariant Produkte (Deutschland) GmbH**
**Patent & License Management Chemicals**
**Industriepark Höchst / G 860**
**65926 Frankfurt am Main (DE)**

(56) References cited:
**WO-A1-2016/164205     US-A1- 2016 184 228**

**Description**

The invention relates to multilamellar vesicles with high fragrance loading and to the use thereof in cosmetic formulations or in laundry applications. In the following the phrase "the invention" is exclusively to be understood and limited as defined in the appended claims.

Many cosmetic and laundry compositions comprise fragrances. These are usually mixed directly into the compositions, for example shampoos, shower gels, face cleansers, solid or liquid soaps or creams and lotions and leave on products in haircare. This procedure has the disadvantage that in most cases upon use only small amounts of the fragrance remain on the skin, on the hair or on the fabric, which can develop their effect there. The majority of the fragrances is usually washed off during use. This leads to large amounts of costly fragrances having to be incorporated into the formulations in order to achieve a desired effect. However, when suitably high amounts of fragrances are used in cosmetic or laundry formulations this may lead to an undesired skin irritation when the formulations are used.

Fragrances are volatile substances. Various approaches of encapsulation have already been used to avoid premature delivery of fragrances. Examples thereof are polymeric encapsulation or inorganic encapsulation. These approaches have been carried out for the development of long-lasting fragrance delivery systems. Polymeric capsules such as melamine formaldehyde, polyacrylates or poly-urethanes usually result in microcapsules with particle size above 1 micron with pressure triggered release in laundry applications. There still exist challenges to encapsulate a broad range of fragrances with different partition coefficients, especially water soluble fragrances. The pressure triggered capsules rather releases the fragrances quickly with friction hence fragrance long-lasting is not prolonged. Diffusion controlled release would be desired for the prolonged fragrance long-lasting in laundry, cosmetics and hair care products.

EP 1 964 544 A1 discloses sensitive skin perfumes. These may be encapsulated within a water insoluble aminoplast capsule.

WO 2008/061384 A1 discloses a batch process for preparation of an emulsion comprising lamellar liquid crystal particles containing fragrance. The process comprises blending the fragrance with emulsifiers capable of forming liquid-crystalline structures, at least one fatty alcohol co-emulsifier having at least 22 carbon atoms, an amphiphilic reinforcing material and a selected wax and adding water slowly to the fragrance mixture thus formed and mixing under shear conditions for obtain a stable emulsion. In the formation of this emulsion a selected surfactant system is used.

US 2007/0105746 A1 discloses compositions for the targeted release of fragrances and aromas. In the encapsulation process a polyol phase A is used in combination with a phase B comprising fragrance, carrier and emulsifier. In the encapsulation process solid lipid nanoparticle dispersions (SLN) are formed. See also WO 2016/164205 A1 (ISP INVESTMENTS INC [US]).

There is still demand for cosmetic or laundry formulations which, on the one hand, allow the amount of fragrances used to be kept low and thus to reduce costs, but which, on the other hand, nevertheless allow a very good effectiveness of the fragrances and providing storage stable formulations.

If the effectiveness of the fragrances could be increased during use, it would be possible to make do with smaller amounts, meaning that the cosmetic or laundry formulations could be produced more cost-effectively.

Surprisingly, it has been found that fragrance molecules over a broad range of partition coefficients ("Log P") can be encapsulated in multilamellar vesicles comprising two or more concentric lipid double layers. These vesicles are sub-micron particles with narrow particle size distribution and with high encapsulation efficiency of fragrance. The encapsulated fragrance is stable under storage conditions and will have long-lasting fragrance release on use thereof.

The present invention relates to multilamellar vesicles in the shape of a rotational body comprising two or more concentric lipid double layers and fragrance, wherein the vesicle has a mean diameter between 100 and 800 nm, the lipid double layers comprise

a) at least one surfactant having a HLB value of greater than 6, and
b) an amphiphilic compound having a log P value of 1 or above, and

wherein the vesicle comprises in addition to components a) and b) a fragrance having a log P value of 1 or above. In the present invention a) and b) are limited as defined in the appended claims.

Surfactant of component a) is characterized by its HLB value of more than 6. The nature of a surfactant is represented by the hydrophilic-lipophilic balance of the molecule. The degree of this hydrophilic-lipophilic balance can be determined by calculating values for the different regions of the molecule, as described by Griffin in 1949 and 1954. Griffin's method has been primarily developed for non-ionic surfactants as described in 1954 works as follows

$$HLB = 20 * M_h / M$$

where $M_h$ is the molecular mass of the hydrophilic portion of the molecule, and M is the molecular mass of the whole molecule, giving a result on a scale of 0 to 20. An HLB value of 0 corresponds to a completely lipophilic molecule, and a value of 20 corresponds to a completely hydrophilic molecule.

The term "HLB" as used in this specification for nonionic surfactants is calculated by the above formula. The method of Griffin is published, for example, in Journal of the Society of Cosmetic Chemists, 5 (4), 249-256 (1954).

The term "HLB" as used in this specification for anionic, cationic or amphoteric surfactants is calculated by the method of Davies. This method is published, for example, in Gas/Liquid and Liquid/Liquid Interfaces. Proceedings of 2nd Inter-national Congress Surface Activity, pp. 426-438, Butterworths, London 1957 Amphiphilic compound of component b) is characterized by its log P value of ≥ 1. The amphiphilic character of component b) can be determined by its partition coefficient between octanol and water. The octanol-water partition coefficient (log P) is a measure of the distribution of a substance between the aqueous and the organic octanol phase and is defined as follows

$$\log P_{\text{oct/wat}} = \log \left( \frac{[\text{solute}]_{\text{octanol}}^{\text{un-ionized}}}{[\text{solute}]_{\text{water}}^{\text{un-ionized}}} \right)$$

Examples of calculated and measured log P values are found in A. Leo, C. Hansch, D. Elkins, Chemical Reviews, Volume 71, no. 6, (1971).

The vesicles of the present invention have a shape of a rotational body, such as that of a sphere or an ellipsoid or that of a disk or other shape of a solid of revolution.

The mean diameter of the vesicles of the present invention is between 80 and 800 nm, preferably between 100 and 500 nm and most preferably between 150 and 400 nm.

The mean diameter is determined by laser diffraction analysis, for example by using a Horiba LA 940 or Mastersizer 3000 from Malvern using the "Mie Scattering Theory" evaluation.

In case of vesicles having axes of different length, such as vesicles having the shape of an ellipsoid or of a disk, the largest axis determines the mean diameter.

The vesicles of the present invention have a narrow particle size distribution of Gaussian shape. Preferably the standard deviation of the particle size distribution is between 10 % and 90 % of the mean diameter.

The vesicles of the present invention contain at least one fragrance having a log P value of 1 or above. The term "log P" has been defined above. The fragrance is an additional component present in the vesicles besides components a) and b).

It has been found that the vesicles of the present invention may incorporate high amounts of one or more fragrances, for example more than 30 % by weight, referring to the total amount of the vesicle. But vesicles having lower amounts of fragrance(s) are also possible.

The vesicles of the present invention may optionally contain co-surfactants as component c) in addition to components a), b) and fragrances. Co-surfactants are surfactants which are not capable of forming micelles. A co-surfactant is any amphiphilic substance having a HLB value ≤6. Preferred co-surfactants have a HLB-value between from 2 to 6.

The vesicles of the present invention may optionally contain waxes as component d) in addition to components a), b) and fragrances or in addition to components a), b), c) and fragrances.

Preferred are vesicles containing fragrances and components a), b) and c).

Preferred are vesicles containing fragrances and components a), b) and d).

Preferred are vesicles containing fragrances and components a), b), c) and d).

The vesicle of the invention comprises several concentric lipid double layers. Although while not being bound by theoretical considerations it is believed that the lipid double layers are arranged in the form of onion shells and that the fragrance molecules are a part of the single lipid double layer composition. As the fragrance molecules can only leave the vesicle via the outer surface, this arrangement provides for the increased storage stability and retarded release of the fragrance from the vesicles.

Due to the buffer systems of the non-fragrance amphiphilic compounds present in the vesicles of the invention the retarded release of different fragrances or fragrance components has similar characteristics as has been demonstrated in the experimental section below.

The multilayer(s) of the vesicles of the present invention can adopt a solid gel phase state at lower temperatures but may undergo phase transition to a fluid state at higher temperatures, and the chemical properties of the amphiphilic compounds constituting such multilayers influence at which temperature this will happen. For controlling the retarded release of the fragrance a solid gel state of the multilayer(s) is preferred. The temperature for the phase transition depends very much on the solidification point of the amphiphilic components in the lipid multilayer(s). The temperature for the phase transition can be determined, for example, by differential scanning calorimetry (DSC).

For the use in fabric softeners a high transition temperature is required, because laundry is often dried at a high heat in a tumble-drier.

Surprisingly, by proper choice of the above mentioned amphiphilic substances a), b), c) and/or d) or other non-fragrance amphiphilics the temperature for the phase transition from a solid gel phase state to a fluid state may be modified within a broad temperature range as has been demonstrated in the experimental section below.

Preferred are vesicles having a phase transition from a solid gel phase state to a fluid state within a range from 30 °C to 90 °C, preferably from 35 °C to 80 °C. This temperature range is preferably chosen for optimum release kinetics and protection of the vesicle against heat.

The lipid double layers of the vesicles of the present invention are formed from a selected combination fragrances with surfactants and amphiphilic compounds defined above as components a) and b) which may optionally contain in addition components c) and/or d).

According to the invention, mixtures of fragrances with selected components a) and b) and optionally with components c) and/or d) are used which form multilamellar liquid-crystalline structures. Such structures can be determined by means of optical microscopy using a polarization microscope. In addition, multilamellar liquid-crystalline structures can be determined by TEM or TEM-freeze fracture technology.

Appropriate techniques are known to the person skilled in the art.

The mixtures of fragrances with components a) and b) and optionally with components c) and/or d) forming the vesicles of this invention are chosen so that a multilamellar liquid-crystalline structure is formed. The selection of suitable amounts of the fragrances and components a) and b) and optionally with components c) and/or d) is possible through simple manual experiments.

The mixtures of fragrances with components a) and b) and optionally with components c) and/or d) forming the vesicles of the invention are chosen so that in water or selected aqueous media multilamellar vesicles are obtainable which have an average diameter of less than 800 nm. The aqueous media maycontain additional additives, such as electrolytes, polyols such as glycerin, polyethylene glycol or propylene glycol, or water soluble vitamins.

According to the invention, the vesicles contain fragrances and components a) and b) and optionally components c) and/or d) which are able to form lyotropic lamellar liquid-crystalline phases. The formation of liquid-crystalline structures is essentially dependent on the geometry of fragrances, components a) and b) and optional components c) and/or d), which can be expressed by the packing parameter PP.

$$PP = V_0 / (a_e * l_0)$$

Vo surfactant tail volume
$a_e$ equilibrium area per molecule at the aggregate interface
lo tail length

A packing parameter PP can be assigned to a chemical species, for example to a surfactant of component a), b), c) or d) or to a fragrance.

If several chemical species are present in certain concentrations to form a mixture of these species, a packing parameter of this mixture $PP_{mixture}$ can be calculated.

A packing parameter of a mixture is defined by the following formula:

$$PP_{mixture} = (\sum c_i * PP_i) / c_{total,}$$

wherein

$PP_i$     is the packing parameter of the single species i,
$c_i$      is the concentration of the single species i in weight percent, and
$c_{total}$     is the total concentration of all i species in the mixture.

Depending on their packing parameter components a) and b) are forming different aggregates. Lyotropic spherical lamellar liquid-crystalline structures as required for entrapping fragrances are formed by components a) and b) optionally in combination with components c) and/or d) at a resulting packing parameter $PP_{mixture}$ of at least 0.5 and preferably in the range between 0.5 and 1.

Compounds with packing parameters PP or $PP_{mixture}$ <0.5 are forming micelles. However, micelles are present in a dynamic equilibrium and continually breakdown and build up again. For this reason, micelles are not very suitable as storage media for other ingredients. As the packing parameter is shifting into a range of 0.3 - 0.5, the compounds or mixtures of compounds form rod-like micelles. Compounds or compound blends with packing parameters > 0.5 - < 1 are forming preferably vesicles. Sandwich double layers are preferably formed at packing parameters around 1. According to the invention, then, components a) and b) and optionally components c) and/or d) are used which may be present in spherical lyotropic lamellar liquid-crystalline phases. In the lyotropic state, fragrance molecules are stored, for example, between the components forming the required vesicle structure. The hydrophilic moiety of a component can be varied according to the desired adhesion to a later substrate. For example, the hydrophilic moiety can be varied for adhesion to the human skin or to textile fibers.

Vesicles of the present invention are preferably formed when the packing parameter of the mixture of all participating surfactants and amphiphilic molecules is 0.5 or above, more preferred in the range 0.5 - 1. This range is valid for spherical vesicles. If the shape of the vesicles is ellipsoid or disk like the packing parameter value shifts to higher values.

HLB values may be correlated to the packing parameter and to Log P values. Therefore it is rather clear that the fragrance molecules encapsulated in the vesicles are a part of the bilayers.

The incorporation of fragrances into the multilamellar liquid-crystalline structure of the vesicles will modify the value of the packing parameter of the mixture of the vesicle loaded with the fragrance.

The packing of lipids within the bilayer also affects its mechanical properties, including its resistance to stretching and bending and including the release kinetics and/or release concentration of the encapsulated fragrances.

Surprisingly the vesicles of the present invention can encapsulate fragrances with a very broad log P range provided components a) and b) and optionally c) and/or d) are present in the multilamellar liquid-crystalline structure.

For matching the required packing parameter of more than about 0.5, preferably of 0.5 to 1.5 and most preferably of 0.5 to 1 for the formation of vesicles with entrapped fragrances components a) and b) and optionally c) and/or d) are required having a sufficient high packing parameter. The used surfactants of component a) can be of nonionic, anionic, cationic or amphoteric structure. Hence it is possible to adapt the surface charge of the vesicles to the surface charge of the application area of the fragrance. This allows a maximum deposition of fragrances.

Surfactants of component a) may be nonionic, anionic, cationic or amphoteric surfactants, provided these have a HLB-value of more than 6.

Examples of suitable nonionic surfactants of component a) are polyoxyethylene sorbitan esters, polyoxyethylene sorbitol esters, polyoxyalkylene fatty alcohol ethers, polyoxyalkylene fatty acid esters, alkoxylated glycerides, polyoxyethylene methyl glucoside ester, alkyl polyglucosides, EO-PO blockpolymers or combinations of two or more thereof.

Examples of anionic surfactants of component a) are sulfonates of alkylbenzenesulfonates, alkanesulfonates, olefinsulfonates, alkyl ether sulfate, alkyl sulfate, sulfosuccinates, alkyl phosphates, alkyl ether phosphates, protein fatty acid condensates, perferably collagen hydrolysates modified with fatty acid, amino acid-based surfactants, isethionates, taurides, acyl lactylates, neutralized fatty acids or combinations of two or more thereof.

Examples of cationic surfactants of component a) are esterquats, ditallow dimethyl ammonium chloride, C12/14 alkyl dimethyl benzyl ammonium chloride, alkyl dimethyl benzil ammonium chloride, cetyl trimethyl ammonium chloride, stearyl trimethyl ammonium chloride, behenyl trimethyl ammonium chloride alkyl hydroxyethyl dimethyl ammonium chloride, distearyl dimethyl ammonium chloride, dihydrogenated tallow fatty alkyl dimethyl ammonium chloride or combinations of two or more thereof.

Examples of amphoteric surfactants of component a) are alkyl amphoacetate, alkyl amidopropyl betaine, alkyl amidopropyl dimethylamine betaine, undecylenamidopropyl betaine, alkyl dimethyl amine oxide

Preferred vesicles of the present invention contain as component a) nonionic surfactants having a HLB-value of more than 6, more preferred polyoxyalkylene fatty alcohol ethers, polyoxyalkylene fatty acid esters, collagen hydrolysates modified with fatty acid or combinations of two or more thereof, most preferred components a) are polyoxyalkylene $C_8$-$C_{24}$-fatty alcohol ethers, polyoxyalkylene $C_8$-$C_{24}$-fatty acid esters, collagen hydrolysates modified with $C_8$-$C_{24}$-fatty acid or combinations of two or more thereof.

Examples of amphiphilic compounds b) are esters of fatty acids, preferagly triglycerides of fatty acids or esters of fatty acids and fatty alcohols or combinations thereof.

Triglycerides of fatty acids or esters of fatty acids and fatty alcohols are amphiphilic components which are not capable of forming micelles. Their amphiphilic character is expressed by the Log P value of 1 or above. For a caprylic/capric acid triglyceride, for example, the Log P is 4.

Examples of tryglycerides of fatty acids are glycerol esters of one or more fatty acids having between 8 and 24 carbon atoms. Examples of esters of fatty acids with fatty alcohols are esters of fatty acids having between 8 and 24 carbon atoms with fatty alcohols having between 8 and 28 carbon atoms. The fatty acid portions of these esters can derived from saturated and/or from ethylenically unsaturated aliphatic fatty acids. Unsaturated fatty acids may have one or more ethylenically unsaturated carbon-carbon bonds. Preferably the triglycerides comprise fatty acid groups from different fatty acids.

Preferred vesicles of the present invention contain triglycerides of one or more fatty acids having 8 to 24 carbon atoms, preferably 10 to 18 carbon atoms, and/or esters of fatty acids having 8 to 24 carbon atoms with fatty alcohols having 8 to 24 carbon atoms, preferably 10 to 18 carbon atoms, as component b).

Examples of suitable co-surfactants of component c) are sorbitan esters, citric esters, lactic esters, partial fatty acid glycerides, polyglycerides, glycerol esters, polyglycerol esters, sorbitol esters, fatty alcohols, propylene glycol esters, methyl glucoside ester, alkyl polyglucosides, sugar esters or combinations of two or more thereof.

Examples of suitable waxes of component d) are waxes of the mono ester type. As waxes are amphiphilic their amphiphilic behavior may be specified by Log P values. Preferrred waxes have Log P values of 4.7 or above, most preferred of 6 or above. As hydrocarbon number increases above C13, as is the case for the majority of the wax constituents, Log P values of 6 ore above are found. Waxes are furthermore characterized by their solidification point, which is typically between 30 and 100 °C. Waxes are organic compounds that characteristically consist of long alkyl chains. They may also include various functional groups such as fatty acids, primary and secondary long chain alcohols, unsaturated bonds, aromatics, amides, ketones, and aldehydes. They frequently contain fatty acid esters as well.

Waxes used as component d) in the present invention can be synthetic ones, animal or plant derived or montan waxes

Optionally, the surfactant mixtures forming the lipid double layers of the vesicles may contain additional polymeric amphiphilic components e) besides the components a), b) and optionally c) and/or d).

Examples of such additional components e) are polymers, such as polyvinyl acetate, polyvinyl pyrrolidone, polyvinyl alcohols, vinyl pyrrolidone (VP)/hexadecene copolymer, VP/ eicosene copolymer or silicone oils and their derivatives. Also these amphiphilic substances can be attributed with a Log P value.

The amount of surfactant(s) of component a) in the lipid double layers of the vesicles of this invention can vary over a broad range. Typical amounts of these surfactant(s) in the lipid double layers may be between 0.1 and 95 % by weight, referring to the total weight of the lipid double layer, preferably between 10 and 40 % by weight.

The amount of amphiphilic components b) in the lipid double layers of the vesicles of this invention can vary over a broad range. Typical amounts of components b) in the lipid double layers may be between 0.1 and 95 % by weight, referring to the total weight of the lipid double layer, preferably between 10 and 40 % by weight.

The amount of co-surfactants, component c), in the lipid double layers of the vesicles of this invention also can vary over a broad range. Typical amounts of co-surfactants in the lipid double layers may be between 0.1 and 50 % by weight, referring to the total weight of the lipid double layer, preferably between 1 and 10 % by weight.

The amount of waxes, component d), in the lipid double layers of the vesicles of this invention also can vary over a broad range. Typical amounts of these waxes in the lipid double layers may be between 0.1 and 50 % by weight, referring to the total weight of the lipid double layer, preferably between 3 and 10 % by weight.

The amount of additional polymeric amphiphilic components e) in the lipid double layers of the vesicles of this invention also can vary over a broad range. Typical amounts of components e) may be between 0.1 and 50 % by weight, referring to the total weight of the lipid double layer, preferably between 3 and 30 % by weight.

In the vesicles of the present invention fragrance molecules with a broad range of LogP, for example in the range of 0.1 - 10, preferably in the range of 1 - 6, can be encapsulated with high encapsulation efficiency.

According to the invention, fragrances are understood as meaning fragrant oils. Basic substances of fragrances are generally essential oils, flower oils, extracts from plant and animal drugs, odorants isolated from natural products, chemically modified (semisynthetic) odorants, and odorants obtained by purely synthetic means.

The fragrances can here originate from a large number of plant starting materials. Examples which may be specified are: flowers, for example from lavender, rose, jasmine, neroli; stems and leaves, for example from geranium, patchouli, petit grain, fruits such as anis, coriander, caroway, juniper; fruit peels, for example from agrumes, such as bergamot, lemon, orange; seeds, such as mace, angelica, celery, cardamom; roots, such as angelica,. costus, iris, calmus; wood, such as sandalwood, guaiac wood, cedar wood, rosewood; herbs and grasses, such as tarragon, lemongrass, sage, thyme; needles and branches, for example from spruce, fir, pine, dwarf-pine; resins and balsams, for example from galvanum, elemi, benzoin, myrrh, olibanum, opoponax.

Animal raw materials are, for example, ambergris, musk, civet, castoreum.

Examples of semisynthetic odorants are isoeugenol, vanillin, hydroxycitronellal, citronellol, geranyl acetate, ionones and methylionones. The completely synthetic odorants or fragrances are very diverse and often orientate themselves to natural substances. For a description of the fragrances, reference may be made, for example, to Römpp, Chemielexikon, 9th edition, keywords "parfums [perfumes]", "riechstoffe [odorants]", "duftstoffe [fragrances]". Further suitable fragrances are known to the person skilled in the art.

The fragrances, for example, can be introduced into the spaces between the hydrophobic moities of the amphiphilic components a), b) and optionally c), d) and/or e), and be stored there. As a result, the fragrances are dissolved, and crystallizing out of the fragrances is prevented. This permits, inter alia, the preparation of cosmetic or laundry formulations with a skin-friendly pH, and by preventing the fragrances from crystallizing out, the skin friendliness of the composition is increased further. The mixtures of amphiphilic components used according to the invention having the fragrances dissolved therein spread upon application to the skin, meaning that application of the fragrance to the skin is improved.

Preferably the vesicles of the present invention are provided in an aqueous composition in an amount of vesicles of from 0.1 to 60 % by weight of the total amount of the composition, preferably between from 1 to 50 % by weight, most preferably between from 5 to 20 % by weight. The aqueous composition may be consisting of only water, water and electrolytes or water and polyols or water and alcohol. The polyols may consist of propylene glycol, polyethylene glycol, glycerin, polyglycerin, sorbitol, isosorbide or dimethyl isosobide.

The vesicles of the present invention may be prepared by feeding the components constituting the vesicles to an emulsification device for manufacture of nano-emulsions. An example of such emulsification device is disclosed in US 2013/0201785 A1.

In this document an emulsifying device for continuous production of emulsions and/or dispersions is disclosed which comprises

a) at least one mixing apparatus comprising a rotationally symmetric chamber sealed airtight on all sides, at least one inlet line for introduction of free-flowing components, at least one outlet line for discharge of the mixed free-flowing components, a stirrer unit which ensures laminar flow and comprises stirrer elements secured on a stirrer shaft, the axis of rotation of which runs along the axis of symmetry of the chamber and the stirrer shaft of which is guided on at least one side, wherein the at least one inlet line is arranged upstream of or below the at least one outlet line, wherein the ratio between the distance between inlet and outlet lines and the diameter of the chamber is $\geq$ 2:1, wherein the ratio between the distance between inlet and outlet lines and the length of the stirrer arms of the stirrer elements is 3:1 to 50:1, and wherein the ratio of the diameter of the stirrer shaft, based on the internal diameter of the chamber, is 0.25 to 0.75 times the internal diameter of the chamber, such that the components introduced into the mixing apparatus via the at least one inlet line are stirred and continuously transported by means of a turbulent mixing area on the inlet side, in which the components are mixed turbulently by the shear forces exerted by the stirrer units, a downstream percolating mixing area in which the components are mixed further and the turbulent flow decreases, a laminar mixing area on the outlet side, in which a lyotropic, liquid-crystalline phase is established in the mixture of the components, in the direction of the outlet line,

b) at least one drive for the stirrer unit and

c) at least one conveying device per component or per component mixture.

The present invention also relates to a method of preparing the vesicles of this invention, said method comprises the steps

i) feeding a composition A comprising at least one surfactant of component a) and water to a first inlet line of an emulsification device,

ii) feeding a composition B comprising at least one amphiphilic compound of component b), fragrance and water to a second inlet line of an emulsification device,

iii) combining compositions A and B in a turbulent mixing zone in the emulsification device,

iv) transporting the mixed compositions within the emulsification device towards an outlet line, whereby laminar flow of the mixed components is established in the zone preceding the outlet line thereby vesicles are formed, and

v) discharging the vesicles via the outlet line form the emulsification device.

In a preferred embodiment of the inventive process the vesicles formed in the emulsification device are diluted with water or an aqueous phase. This can be performed in a separate device by introducing the vesicles into water which optionally contains additional surfactants. The aqueous composition may be consisting of only water, water and electrolytes or water and polyols. The polyols may consist of propylenglycol, polyethylene glycol, glycerin, polyglycerin, sorbitol, isosorbide or dimethyl isosobide.

The vesicles of the present invention may be preferably used in cosmetic formulations or in laundry formulations.

Cosmetic formulations are preferably skin-treatment compositions or hair-treatment compositions.

Laundry formulations are preferably laundry additives, washing agents or fabric softeners.

Cosmetic formulations or laundry formulations usually comprise further ingredients typical of these formulations. The combination of fragrance-containing vesicles, water and optionally other substances can, however, also like the vesicles and water itself, be used for producing hair- and/or skin-cleansing compositions or for producing washing agents and/or fabric softeners. Such hair- and/or skin-cleansing compositions may be present in any desired suitable form, for example as shampoos, shower gels, face cleansers or soaps.

Such or washing agents or fabric softeners may be present in any desired suitable form, for example as powders or concentrates.

Over and above the storage effect, the vesicles of to the invention permit also extensive protection of the fragrances against oxidative decomposition. If appropriate, further antioxidants can also be added.

Even without the addition of antioxidants, the fragrances in the vesicles or in the cosmetic or laundry compositions according to the invention is significantly better protected against oxidation than in conventional application forms.

The invention also relates to the use of the vesicles described above in cosmetic compositions, preferably in compositions for skin treatment or for hair treatment.

The invention furthermore relates to the use of the vesicles described above in laundry compositions, preferably in washing agents or in fabric softeners.

The invention is illustrated in more detail by the examples below.

EXAMPLES

Example 1

phase A:

2.5 % b.wt. ethoxylated lauryl alcohol ethoxylation degree 23 (Brij L23)
2.5 % b.wt. ethoxylated stearyl alcohol ethoxylation degree 100 (Brij S100)
2.5 % b.wt. glycerine
2.5 % b.wt. water

phase B:

10 % b.wt. cetyl palmitate (Cutina CP)
10 % b.wt. triglyceride of caprylic acid / capric acid (Rotefan CCT)
20 % b.wt. fragrance

phase C:

0.05 % b.wt. methylchloroisothiazolinone (Kathon CG)
49.5 % b.wt. water

The ingredients of phase A and of phase B are introduced into the inlet lines of an emulsification device disclosed in US 2013/0201785 A1. The product from this emulsification device is a composition consisting essentially of multilamellar vesicles in the shape of a sphere comprising two or more concentric lipid double layers and fragrance. The fragrance loading is more than 50 % by weight, referred to the total weight of the vesicle.

The product obtained from the emulsification device is introduced under stiffing into a vessel containing phase C. An aqueous composition comprising multilamellar vesiclesis formed.

For example 1 the average particle size measured with Horiba LA 940 is 164 nm with standard deviation 49 nm.

Example 2

phase A:

2 % b.wt. collagen hydrolysate sodium salt modified with lauric acid (LameponS)
2 % b.wt. ethoxylated lauryl alcohol ethoxylation degree 23 (Brij L23)
2 % b.wt. ethoxylated stearyl alcohol ethoxylation degree 100 (Brij S100)
2.5 % b.wt. glycerine
1.1 % b.wt. water

phase B:

5 % b.wt. cetyl palmitate (Cutina CP)
10 % b.wt. triglyceride of caprylic acid / capric acid (Rotefan CCT)
5 % b.wt. polyvinylacetate (Dodiflow 5599)
20 % b.wt. fragrance

phase C:

0.05 % b.wt. methylchloroisothiazolinone (Kathon CG)
49.5 % b.wt. water

The compositions of phases A and B are processed as in Example 1 and the obtained product is diluted with phase C as described in Example 1.

For example 2 the average particle size measured with Horiba LA 940 is 173 nm with standard deviation 53 nm.

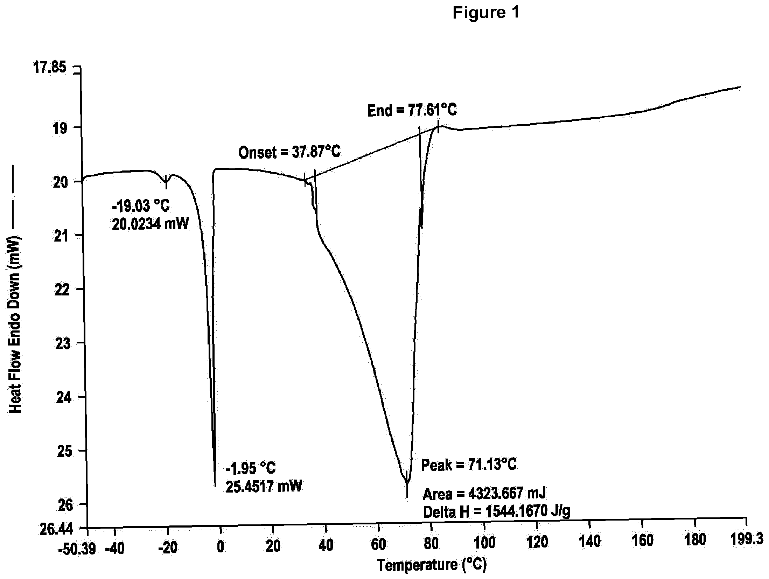

Further formation of multilamellar vesicle have been characterized with DSC (differential scanning calorimetry). The results are shown in Figure 1. The sharp endothermic peak in the DSC curve of Figure 1 confirms the transition of the vesicles from a solid gel phase state to a fluid state.

Figure 1 demonstrates that the proper selection of amphiphilic substances, such as monoester waxes, triglycerides or other non fragrance amphiphilics transform the temperature of the phase transition into a broad temperature range. Here the transition temperature covers a range from 38 °C to 78 °C.

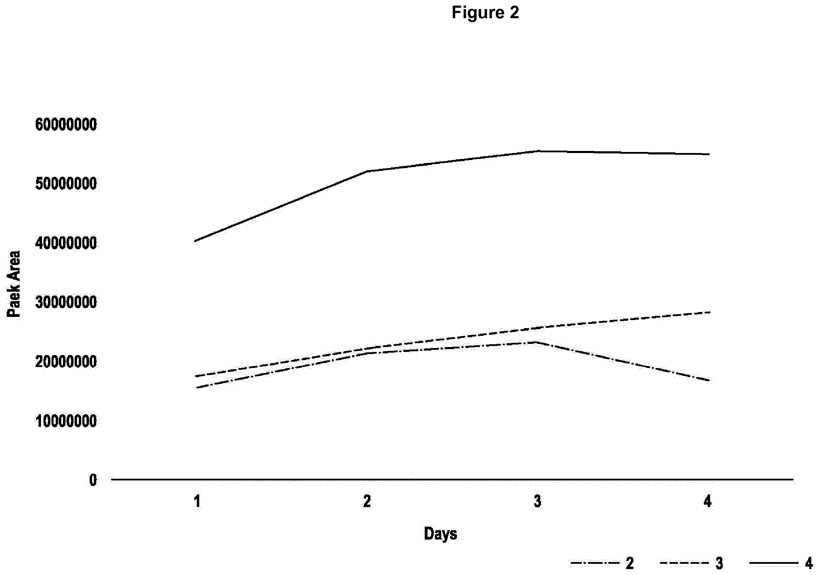

Encapsulated fragrance samples prepared in analogy to example 2 are diluted at 10 % in water and a cotton swatch has been exposed in these diluted samples. After absorption of the fragrance encapsulated samples on the swatch they were dried at room temperature and then the samples were evaluated for release in a microchamber at controlled temperature of 80 °C for 20 minutes. The samples desorbed at 300 °C for 10 minutes and measured were with GCMS for each day. The graph of Figure 2 shows the fragrance is releasing in controlled manner.

The curves 2,3 and 4 of the fragrance ingredients shown in Figure 2 are mentioned below:

2 4-ter.-butyl cyclohexyl acetate (log P = 3.96)

3 alpha-isomethyl ionone (log P = 4.41)

4 Lilal (log P = 2.00)

These curves demonstrate that the release characteristics of the fragrances is not dependent upon the log P value of the fragrance.

Example 3 (with higher fragrance amount in composition)

| Phase A | % wt |
|---|---|
| ethoxylated lauryl alcohol ethoxylation degree 23 (Brij L23) | 3.50 |
| ethoxylated stearyl alcohol ethoxylation degree 100 (Brij S100) | 3.50 |
| Glycerine | 2.50 |
| Water | 4.00 |
| Phase B | |
| cetyl palmitate (Cutina CP) | 7.50 |
| triglyceride of caprylic acid / capric acid (Rotefan CCT) | 7.50 |
| polyvinylacetate (Dodiflow 5599) | 7.50 |
| fragrance | 37.50 |

(continued)

| Phase C | |
|---|---|
| Water | 26.50 |
| Total : | 100.00 |

For example 3 the average particle size measured with Horiba LA 940 is 184 nm with standard deviation 69 nm.

Example 4 (with increase in particle size)

| Phase A | % wt |
|---|---|
| ethoxylated lauryl alcohol ethoxylation degree 23 (Brij L23) | 2.50 |
| ethoxylated stearyl alcohol ethoxylation degree 100 (Brij S100) | 2.50 |
| water | 3.00 |
| Phase B | |
| cetyl palmitate (Cutina CP) | 20.00 |
| fragrance | 20.00 |
| Phase C | |
| water | 41.50 |
| glycerine | 10.00 |
| phenoxyethanol | 0.50 |
| Total : | 100.00 |

For example 4 the average particle size measured with Horiba LA 940 is 259 nm with standard deviation 58 nm.

Example 5 (with cationic surfactant)

| Phase A | % wt |
|---|---|
| ethoxylated lauryl alcohol ethoxylation degree 23 (Brij L23) | 2.50 |
| ethoxylated stearyl alcohol ethoxylation degree 100 (Brij S100) | 2.50 |
| alkyl amines (Genamin CTAC) | 2.00 |
| glycerine | 2.50 |
| water | 4.00 |
| Phase B | |
| cetyl palmitate (Cutina CP) | 7.50 |
| triglyceride of caprylic acid / capric acid (Rotefan CCT) | 7.50 |
| polyvinylacetate (Dodiflow 5599) | 7.50 |
| fragrance | 37.50 |
| Phase C | |
| water | 26.50 |
| Total : | 100.00 |

For example 5 the average particle size measured with Horiba LA 940 is 170 nm with standard deviation 68 nm.

Example 6 (formulation of a fabric softener)

| Name of ingredients | Function | % |
|---|---|---|
| Propagen TQSV-IPA (Triethanolamine Esterquat) | cation ic softener | 8 |
| distilled water | vehicle | up to 100 |
| colour [Sanoline Blue AE90 /9 0.1 5)] | color | 3 |
| encapsulated fragrance nanocon sample | smell | 0.3-10% |
| Perlogen 3000 (glycol distearate, laureth-4 cocamidopropyl betaine) | sheen & shine | 1 |
| Genapol LT (PEG-150 polyglyceryl-2 tristearate and laureth-3 and dipropylene glycol) | liquid thickner | 0.5 |

Example 7 (personal care cleansing formulation)

| Name of ingredient | % |
|---|---|
| cocoamide DEA or MEA | 5-10 |
| alcohol ethoxy glyceryl sulfonate | 2-25 |
| sodium or ammonium lauryl sulfate | 5-20 |
| cocoamidopropyl betaine | 0-20 |
| polysorbates (Tween 20, 40, 60) | 0-5 |
| encapsulated fragrance nanocon sample | 0.3-10 |

**Claims**

1. A multilamellar vesicle in the shape of a rotational body comprising two or more concentric lipid double layers and fragrance wherein the vesicle has a mean diameter between 100 and 800 nm, the lipid double layers comprise

   a) at least one surfactant having a HLB value of greater than 6, selected from group consisting of polyoxyalkylene $C_8$-$C_{24}$-fatty alcohol ethers, polyoxyalkylene $C_8$-$C_{24}$-fatty acid esters, collagen hydrolysates modified with $C_8$-$C_{24}$-fatty acid or combinations of two or more thereof, and
   b) an amphiphilic compound having a logP value of 1 or above, selected from the group consisting of triglycerides of $C_8$-$C_{24}$-fatty acids and esters of $C_8$-$C_{24}$-fatty acids and $C_8$-$C_{24}$-fatty alcohols or combinations thereof, and

   wherein the vesicle comprises in addition to components a) and b) a fragrance having a logP value of 1 or above.

2. The vesicle according to claim 1, wherein the shape of the vesicle is spherical, ellipsoidal or disk-like.

3. The vesicle according to claim 2, wherein the mean diameter of the vesicle is between 100 and 500 nm and wherein the size distribution of the vesicles is Gaussian having a standard deviation between of 10 and 90 % of the mean diameter.

4. The vesicle according to claim 3, wherein the mean diameter of the vesicle is between 100 and 200 nm.

5. The vesicle according to at least one of the claims 1 to 4, wherein the vesicles contains at least one co-surfactant as component c) in addition to components a), b) and fragrance.

6. The vesicle according to at least one of the claims 1 to 5, wherein the vesicle contains at least one wax as component d) in addition to components a), b) and fragrance or in addition to components a), b), c) and fragrance.

7. The vesicle according to claims 5 and 6, wherein the vesicle contains fragrance and components a), b), c) and d).

8. The vesicle according to at least one of the claims 1 to 7, wherein the vesicle comprises components a), b) and optionally component c) and/or component d) and wherein the packing parameter of the mixture of components a), b) and optionally component c) and/or component d) has a value of 0.5 or above, more preferred in the range of 0.5 to 1.

9. The vesicle according to at least one of the claims 5 to 8, wherein the co-surfactant is selected from the group consisting of sorbitan esters, citric esters, lactic esters, partial fatty acid glycerides, polyglycerides, glycerol esters, polyglycerol esters, sorbitol esters, fatty alcohols, propylene glycol esters, methyl glucoside ester, alkyl polyglucosides, sugar esters or combinations of two or more thereof.

10. The vesicle according to at least one of the claims 6 to 8, wherein component d) is a wax of the mono ester type.

11. The vesicle according to at least one of the claims 1 to 10, wherein the vesicle contains as component e) at least one amphiphilic copolymer, preferably polyvinyl acetate, polyvinyl pyrrolidone, polyvinyl alcohols, vinyl pyrrolidone/-hexadecene copolymer, vinyl pyrrolidone/eicosene copolymer, silicone oils or their derivatives.

12. The vesicle according to at least one of the claims 1 to 11, wherein the fragrance has a log P value in the range between 1 and 10.

13. An aqueous composition comprising the vesicles according to at least one of the claims 1 to 12 and water, wherein the amount of vesicles is 0.1 to 60 % by weight of the total amount of the composition.

14. The composition according to claim 13, wherein the amount of the vesicles is 1 to 50 % by weight, preferably 5 to 20 % by weight of the total amount of the composition.

15. A method of manufacturing the vesicles according to at least one of the claims 1 to 12 comprising the steps:

> i) feeding a composition A comprising at least one surfactant of component a), selected from group consisting of polyoxyalkylene $C_8$-$C_{24}$-fatty alcohol ethers, polyoxyalkylene $C_8$-$C_{24}$-fatty acid esters, collagen hydrolysates modified with $C_8$-$C_{24}$-fatty acid or combinations of two or more thereof, and water to a first inlet line of an emulsification device,
> ii) feeding a composition B comprising at least one amphiphilic compound of component b), selected from the group consisting of triglycerides of $C_8$-$C_{24}$-fatty acids and esters of $C_8$-$C_{24}$-fatty acids and $C_8$-$C_{24}$-fatty alcohols or combinations thereof, and fragance and water to a second inlet line of an emulsification device,
> iii) combining compositions A and B in a turbulent mixing zone in the emulsification device,
> iv) transporting the mixed compositions within the emulsification device towards an outlet line, whereby laminar flow of the mixed components is established in the zone preceding the outlet line thereby vesicles are formed, and
> v) discharging the vesicles via the outlet line form the emulsification device.

16. The method according to claim 15, wherein the vesicles formed in the emulsification device are diluted with water by introducing the vesicles into water which optionally contains additional surfactants into a separate device.

17. Use of the vesicles according to at least one of the claims 1 to 12 in cosmetic and hair care compositions.

18. Use of the vesicles according to at least one of the claims 1 to 12 in laundry compositions, preferably in washing agents and in fabric softeners.

19. The vesicle according to at least one of the claims 1 to 12 providing prolonged fragrance release by slow diffusion in laundry, cosmetics and hair care products.

**Patentansprüche**

1. Multilamellare Vesikel in Form eines Rotationskörpers, umfassend zwei oder mehr konzentrische Lipiddoppelschichten und Duftstoff, wobei die Vesikel einen mittleren Durchmesser zwischen 100 und 800 nm aufweist und die Lipiddoppelschichten umfassen:

> a) mindestens ein Tensid mit einem HLB-Wert größer als 6 ausgewählt aus der Gruppe bestehend aus Polyoxyalkylen-$C_8$-$C_{24}$-fettalkoholethern, Polyoxy-alkylen-$C_8$-$C_{24}$-fettsäureestern, Kollagenhydrolysaten, die mit

EP 3 498 253 B1

$C_8$-$C_{24}$-Fettsäure modifiziert sind, oder Kombinationen von zwei oder mehr davon, und
b) eine amphiphile Verbindung mit einem logP-Wert von 1 oder darüber ausgewählt aus der Gruppe bestehend aus Triglyceriden von $C_8$-$C_{24}$-Fettsäuren und Estern von $C_8$-$C_{24}$-Fettsäuren und $C_8$-$C_{24}$-Fettalkoholen oder Kombinationen davon, und

wobei die Vesikel zusätzlich zu den Komponenten a) und b) einen Duftstoff mit einem logP-Wert von 1 oder darüber umfasst.

2. Vesikel nach Anspruch 1, wobei die Form der Vesikel sphärisch, elliptisch oder scheibenartig ist.

3. Vesikel nach Anspruch 2, wobei der mittlere Durchmesser der Vesikel zwischen 100 und 500 nm liegt, und wobei die Größenverteilung der Vesikel eine Gauß-Verteilung mit einer Standardabweichung zwischen 10 und 90 % des mittleren Durchmessers ist.

4. Vesikel nach Anspruch 3, wobei der mittlere Durchmesser der Vesikel zwischen 100 und 200 nm liegt.

5. Vesikel nach mindestens einem der Ansprüche 1 bis 4, wobei die Vesikel mindestens ein Cotensid als Komponente c) zusätzlich zu den Komponenten a), b) und Duftstoff enthält.

6. Vesikel nach mindestens einem der Ansprüche 1 bis 5, wobei die Vesikel mindestens ein Wachs als Komponente d) zusätzlich zu den Komponenten a), b) und Duftstoff oder zusätzlich zu den Komponenten a), b), c) und Duftstoff enthält.

7. Vesikel nach den Ansprüchen 5 und 6, wobei die Vesikel Duftstoff und Komponenten a), b), c) und d) enthält.

8. Vesikel nach mindestens einem der Ansprüche 1 bis 7, wobei die Vesikel Komponenten a), b) und gegebenenfalls Komponente c) und/oder Komponente d) umfasst, und wobei der Packparameter der Mischung von Komponenten a), b) und gegebenenfalls Komponente c) und/oder Komponente d) einen Wert von 0,5 oder darüber aufweist, bevorzugter im Bereich von 0,5 bis 1.

9. Vesikel nach mindestens einem der Ansprüche 5 bis 8, wobei das Cotensid ausgewählt ist aus der Gruppe bestehend aus Sorbitanestern, Citronensäureestern, Milchsäureestern, partiellen Fettsäureglyceriden, Polyglyceriden, Glycerinestern, Polyglycerinestern, Sorbitolestern, Fettalkoholen, Propylenglykolestern, Methylglucosidester, Alkylpolyglucosiden, Zuckerestern oder Kombinationen von zwei oder mehr davon.

10. Vesikel nach mindestens einem der Ansprüche 6 bis 8, wobei Komponente d) ein Wachs vom Monoestertyp ist.

11. Vesikel nach mindestens einem der Ansprüche 1 bis 10, wobei die Vesikel als Komponente e) mindestens ein amphiphiles Copolymer, vorzugsweise Polyvinylacetat, Polyvinylpyrrolidon, Polyvinylalkohole, Vinylpyrrolidon/Hexadecen-Copolymer, Vinylpyrrolidon/Eicosen-Copolymer, Silikonöle oder deren Derivate enthält.

12. Vesikel nach mindestens einem der Ansprüche 1 bis 11, wobei der Duftstoff einen logP-Wert im Bereich von 1 bis 10 aufweist.

13. Wässrige Zusammensetzung, umfassend die Vesikel nach mindestens einem der Ansprüche 1 bis 12 und Wasser, wobei die Menge der Vesikel 0,1 bis 60 Gew.% der Gesamtmenge der Zusammensetzung beträgt.

14. Zusammensetzung nach Anspruch 13, wobei die Menge der Vesikel 1 bis 50 Gew.%, vorzugsweise 5 bis 20 Gew.% der Gesamtmenge der Zusammensetzung beträgt.

15. Verfahren zur Fertigung der Vesikel nach mindestens einem der Ansprüche 1 bis 12, umfassend die Schritte:

i) Einspeisen einer Zusammensetzung A, umfassend mindestens ein Tensid von Komponente a) ausgewählt aus der Gruppe bestehend aus Polyoxyalkylen-$C_8$-$C_{24}$-fettalkoholethern, Polyoxyalkylen-$C_8$-$C_{24}$-fettsäureestern, Kollagenhydrolysaten, die mit $C_8$-$C_{24}$-Fettsäure modifiziert sind, oder Kombinationen von zwei oder mehr davon, und Wasser in eine erste Einlassleitung einer Emulgiervorrichtung,
ii) Einspeisen einer Zusammensetzung B, umfassend mindestens eine amphiphile Verbindung von Komponente b) ausgewählt aus der Gruppe bestehend aus Triglyceriden von $C_8$-$C_{24}$-Fettsäuren und Estern von $C_8$-$C_{24}$-Fett-

13

säuren und $C_8$-$C_{24}$-Fettalkoholen oder Kombinationen davon, und Duftstoff und Wasser in eine zweite Einlassleitung einer Emulgiervorrichtung,

iii) Kombinieren der Zusammensetzungen A und B in einer turbulenten Mischzone in der Emulgiervorrichtung,

iv) Transportieren der gemischten Zusammensetzungen innerhalb der Emulgiervorrichtung in Richtung einer Auslassleitung, wobei laminare Strömung der gemischten Komponenten in der Zone hergestellt wird, welche der Auslassleitung vorangeht, wodurch Vesikel gebildet werden, und

v) Austragen der Vesikel über die Auslassleitung aus der Emulgiervorrichtung.

16. Verfahren nach Anspruch 15, wobei die in der Emulgiervorrichtung gebildeten Vesikel mit Wasser verdünnt werden, indem die Vesikel in Wasser, das gegebenenfalls zusätzliche Tenside enthält, in einer separaten Vorrichtung eingebracht werden.

17. Verwendung der Vesikel nach mindestens einem der Ansprüche 1 bis 12 in kosmetischen und Haarpflegezusammensetzungen.

18. Verwendung der Vesikel nach mindestens einem der Ansprüche 1 bis 12 in Zusammensetzungen zur Wäschebehandlung, vorzugsweise in Waschmitteln und in Weichspülern.

19. Vesikel nach mindestens einem der Ansprüche 1 bis 12, das verlängerte Duftstofffreisetzung durch langsame Diffusion in Wäschebehandlungs-, kosmetischen und Haarpflegeprodukten bereitstellt.


## Revendications

1. Vésicule multilamellaire sous la forme d'un corps de rotation comprenant deux, ou plus, doubles couches lipidiques concentriques et une fragrance, la vésicule possédant un diamètre moyen compris entre 100 et 800 nm, les doubles couches lipidiques comprenant

   a) au moins un agent tensioactif ayant une valeur HLB supérieure à 6, choisi dans le groupe constitué par les éthers d'alcools gras en $C_8$-$C_{24}$ polyoxyalkylénés, les esters d'acides gras en $C_8$-$C_{24}$ polyoxyalkylénés, les hydrolysats de collagène modifiés par des acides gras en $C_8$-$C_{24}$, ou des combinaisons de deux, ou plus, parmi ceux-ci, et
   b) un composé amphiphile ayant une valeur de log P de 1 ou plus, choisi dans le groupe constitué par les triglycérides d'acides gras en $C_8$-$C_{24}$, et les esters d'acides gras en $C_8$-$C_{24}$ et les alcools gras en $C_8$-$C_{24}$, ou des combinaisons de ceux-ci, et

   la vésicule comprenant, outre les composants a) et b), une fragrance ayant une valeur de log P de 1 ou plus.

2. Vésicule selon la revendication 1, la forme de la vésicule étant sphérique, ellipsoïdale ou en forme de disque.

3. Vésicule selon la revendication 2, le diamètre moyen de la vésicule étant compris entre 100 et 500 nm, et où la distribution granulométrique des vésicules est gaussienne, ayant un écart-type compris entre 10 et 90% du diamètre moyen.

4. Vésicule selon la revendication 3, le diamètre moyen de la vésicule étant compris entre 100 et 200 nm.

5. Vésicule selon au moins l'une des revendications 1 à 4, la vésicule contenant au moins un co-agent tensioactif comme composant c), outre les composants a), b) et la fragrance.

6. Vésicule selon au moins l'une des revendications 1 à 5, la vésicule contenant au moins une cire comme composant d), outre les composants a), b) et la fragrance, ou outre les composants a), b), c) et la fragrance.

7. Vésicule selon les revendications 5 et 6, la vésicule contenant une fragrance et les composants a), b), c) et d).

8. Vésicule selon au moins l'une des revendications 1 à 7, la vésicule comprenant les composants a), b) et éventuellement le composant c) et/ou le composant d) et où le paramètre de tassement du mélange des composants a), b) et éventuellement du composant c) et/ou du composant d) possède une valeur de 0,5 ou plus, plus préférablement dans la plage allant de 0,5 à 1.

**9.** Vésicule selon au moins l'une des revendications 5 à 8, dans laquelle le co-agent tensioactif est choisi dans le groupe constitué par les esters de sorbitane, les esters citriques, les esters lactiques, les glycérides d'acides gras partiels, les polyglycérides, les esters de glycérol, les esters de polyglycérol, les esters de sorbitol, les alcools gras, les esters de propylène glycol, un ester de glucoside de méthyle, les polyglucosides d'alkyle, les esters de sucre ou des combinaisons de deux, ou plus, parmi ceux-ci.

**10.** Vésicule selon au moins l'une des revendications 6 à 8, dans laquelle le composant d) est une cire de type monoester.

**11.** Vésicule selon au moins l'une des revendications 1 à 10, la vésicule contenant, comme composant e), au moins un copolymère amphiphile, préférablement l'acétate de polyvinyle, la polyvinylpyrrolidone, les alcools polyvinyliques, un copolymère de vinylpyrrolidone/ hexadécène, un copolymère de vinylpyrrolidone/éicosène, des huiles de silicone ou leurs dérivés.

**12.** Vésicule selon au moins l'une des revendications 1 à 11, dans laquelle la fragrance possède une valeur de log P dans la plage comprise entre 1 et 10.

**13.** Composition aqueuse comprenant les vésicules selon au moins l'une des revendications 1 à 12 et de l'eau, dans laquelle la quantité de vésicules va de 0,1 à 60% en poids de la quantité totale de la composition.

**14.** Composition selon la revendication 13, dans laquelle la quantité de vésicules va de 1 à 50% en poids, préférablement de 5 à 20% en poids, de la quantité totale de la composition.

**15.** Méthode de préparation des vésicules selon au moins l'une des revendications 1 à 12, comprenant les étapes

i) d'alimentation d'une composition A comprenant au moins un agent tensioactif à base de composant a) choisi dans le groupe constitué par les éthers d'alcools gras en $C_8$-$C_{24}$ polyoxyalkylénés, les esters d'acides gras en $C_8$-$C_{24}$ polyoxyalkylénés, les hydrolysats de collagène modifiés par des acides gras en $C_8$-$C_{24}$, ou des combinaisons de deux, ou plus, parmi ceux-ci, et d'eau, dans une première conduite d'entrée d'un dispositif d'émulsification,

ii) d'alimentation d'une composition B comprenant au moins un composé amphiphile à base de composant b) choisi dans le groupe constitué par les triglycérides d'acides gras en $C_8$-$C_{24}$, et les esters d'acides gras en $C_8$-$C_{24}$ et les alcools gras en $C_8$-$C_{24}$, ou des combinaisons de ceux-ci, et d'une fragrance et d'eau, dans une deuxième conduite d'entrée d'un dispositif d'émulsification,

iii) de combinaison des compositions A et B dans une zone de mélange turbulent dans le dispositif d'émulsification,

iv) de transport des compositions mélangées au sein du dispositif d'émulsification vers une conduite de sortie, ce par quoi un flux laminaire des composants mélangés est établi dans la zone précédant la conduite de sortie, formant ainsi les vésicules, et

v) de décharge des vésicules via la conduite de sortie à partir du dispositif d'émulsification.

**16.** Méthode selon la revendication 15, dans laquelle les vésicules formées dans le dispositif d'émulsification sont diluées par de l'eau par l'introduction des vésicules dans de l'eau qui contient éventuellement des agents tensioactifs supplémentaires dans un dispositif séparé.

**17.** Utilisation des vésicules selon au moins l'une des revendications 1 à 12, dans des compositions cosmétiques et de soin des cheveux.

**18.** Utilisation des vésicules selon au moins l'une des revendications 1 à 12, dans des compositions de lessive, préférablement dans des agents de lavage et dans des adoucissants pour le linge.

**19.** Vésicule selon au moins l'une des revendications 1 à 12, fournissant une libération prolongée de fragrance par diffusion lente dans des produits de lessive, cosmétiques et de soin des cheveux.

Figure 1

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1964544 A1
- WO 2008061384 A1
- US 20070105746 A1
- WO 2016164205 A1
- US 20130201785 A1

### Non-patent literature cited in the description

- *Journal of the Society of Cosmetic Chemists,* 1954, vol. 5 (4), 249-256
- Gas/Liquid and Liquid/Liquid Interfaces. Proceedings of 2nd Inter-national Congress Surface Activity. Butterworths, 1957, 426-438
- **A. LEO ; C. HANSCH ; D. ELKINS.** *Chemical Reviews,* 1971, vol. 71 (6